Europäisches Patentamt

European Patent Office

Office européen des brevets

(11) Veröffentlichungsnummer: **0 298 234 B1**

## (12) EUROPÄISCHE PATENTSCHRIFT

(45) Veröffentlichungstag der Patentschrift: **01.04.92**

(21) Anmeldenummer: **88108215.0**

(22) Anmeldetag: **21.05.88**

(51) Int. Cl.⁵: **A61F 2/34**

---

(54) **Hüftgelenkspfanne.**

---

(30) Priorität: **09.07.87 CH 2607/87**

(43) Veröffentlichungstag der Anmeldung:
**11.01.89 Patentblatt 89/02**

(45) Bekanntmachung des Hinweises auf die
Patenterteilung:
**01.04.92 Patentblatt 92/14**

(84) Benannte Vertragsstaaten:
**AT DE FR GB IT**

(56) Entgegenhaltungen:
**EP-A- 0 066 092**
**DE-A- 3 602 081**
**US-A- 3 806 960**

(73) Patentinhaber: **GEBRÜDER SULZER AKTIENGE-
SELLSCHAFT**
**Zürcherstrasse 9**
**CH-8401 Winterthur(CH)**

(72) Erfinder: **Frey, Otto**
**Wallrütistrasse 56**
**CH-8400 Winterthur(CH)**
Erfinder: **Koch, Rudolf**
**Oberdorfstrasse 229**
**CH-8267 Berlingen(CH)**

## Beschreibung

Die Erfindung betrifft eine Hüftgelenkspfanne mit einer Aussenschale aus einem wenig verformbaren Werkstoff, in der ein ebenfalls wenig verformbarer Pfannenkörper für die Aufnahme des Gelenkkopfes einer Femurkopfprothese durch lösbar verankert ist, daß am äusseren Umfang des Pfannenkörpers in einer Nut ein einfach geschlitzter Ring eingelegt ist, der mit einer aus seinem Umfang vorspringenden ringförmigen Nase in eine Ringnut in der Innenwandung der Aussenschale einschnappt. Unter "wenig verformbaren Werkstoffen" werden dabei Metalle, Keramik oder auch faserverstärkte Kunststoffe verstanden, bei denen Aussenschale und Pfannenkörper zur gegenseitigen Montage nicht soweit relativ zueinander verformt werden können, dass sie beispielsweise über einen Schnappverschluss formschlüssig und lösbar miteinander verbunden werden können.

Eine derartige Hüftgelenkspfanne ist in der EP-A-0 066 092 beschrieben.

Werkstoffe, die die unterschiedlichen, an eine Hüftgelenkspfanne zu stellenden Forderungen optimal erfüllen, sind bisher nicht bekannt. Man ist daher für die Einhaltung optimaler Bedingungen - beispielsweise für die Körperverträglichkeit auf der einen Seite und für ein gutes tribologisches Verhalten auf der anderen Seite - gezwungen, Aussenschale und Pfannenkörper aus verschiedenen Werkstoffen herzustellen. Bei einer Pfanne mit den genannten Eigenschaften ist die Aussenschale beispielsweise aus Titan oder einer Titanlegierung, während der Pfannenkörper aus einer CoCrMo-Gusslegierung besteht.

Die Patentanmeldung EP-A-0 066 092 zeigt die Verbindung einer Aussenschale mit einer Innenschale über eine elastische Pufferschicht und eine Sicherung der Verbindung durch eine C-förmige Spreizfeder, die vorzugsweise aus Metall besteht. Mit dieser Lösung lässt sich zwischen den beiden Schalen Abrieb und dessen Herausschwemmen durch die mit der Relativbewegung verbundene Pumpwirkung nicht verhindern.

Aufgabe der Erfindung ist es, Aussenschale und Pfannenkörper auf einfache Weise relativ zueinander unbeweglich, jedoch lösbar, miteinander zu verbinden. Erfindungsgemäss besteht die Lösung darin, dass der Ring aus Kunststoff ist und sich vollumfänglich auf dem Grund der Nut des Pfannenkörpers abstützt, und dass Aussenschale und Pfannenkörper miteinander formschlüssig verbunden sind.

Die Elastizität des Kunststoffringes ermöglicht den Einsatz der bewährten Verbindung von Aussenschale und Pfannenkörper mittels eines Schnappverschlusses für den Fall, dass beide Elemente aus wenig verformbarem Werkstoff sind. Die

Demontage der Innenschale erfolgt durch Zerstören des Kunststoffringes.

Als vorteilhaft für die Montage des Pfannenkörpers in der bereits implantierten und im Knochen fixierten Aussenschale hat es sich erwiesen, wenn Pfannenkörper und Aussenschale mit zylindrischen Führungen versehen sind, die dem Ring polseitig vorgelagert sind. Weiterhin ist es zweckmässig, die beiden Elemente der Hüftgelenkspfanne gegen Verdrehung relativ zueinander zu sichern.

Im folgenden wird die Erfindung anhand eines Ausführungsbeispiels im Zusammenhang mit der Zeichnung näher erläutert.

Fig. 1  stellt rein schematisch eine zweiteilige Hüftgelenkspfanne in einem Meridianschnitt dar;

Fig. 2  ist ein vergrössertes Detail aus Fig. 1;

Fig. 3  ist eine Aufsicht auf den Kunststoffring, der beide Pfannenteile miteinander verbindet.

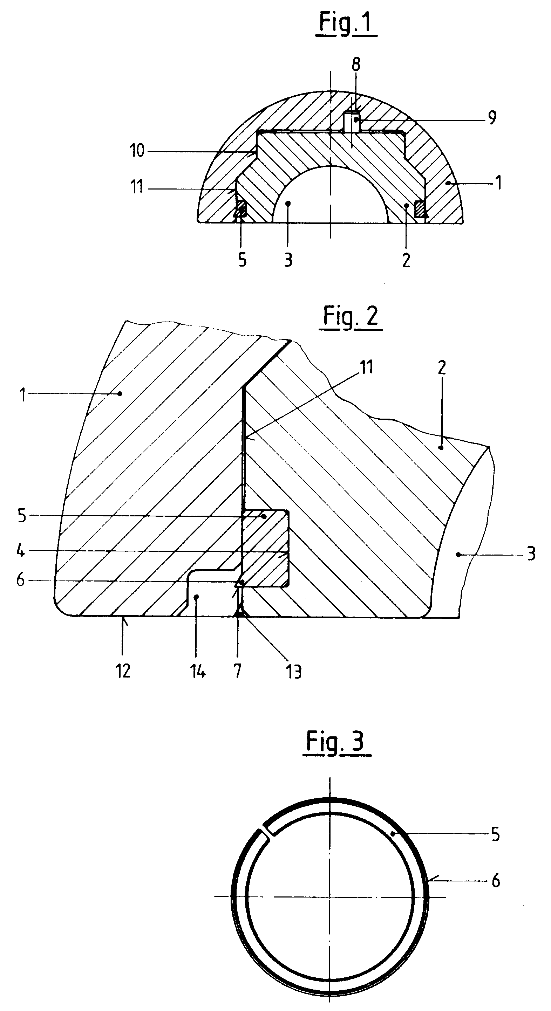

Wie bereits erwähnt, besteht die stark schematisiert dargestellte Aussenschale 1 (Fig. 1) der neuen Hüftgelenkspfanne aus Reintitan oder einer Titanlegierung, da sie in relativ grossflächigem Kontakt mit Körperbewebe steht. In sie ist ein Pfannenkörper 2 eingesetzt, der die eigentliche Pfannenschale 3 zur Aufnahme eines nicht dargestellten Gelenkkopfes einer Femurkopfprothese enthält. Da vom Werkstoff des Pfannenkörpers 2 vor allem gute tribologische Eigenschaften gefordert werden, besteht dieser beispielsweise aus einer CoCrMo-Gusslegierung bekannter Zusammensetzung.

In eine Nut 4 am äusseren Umfang des Pfannenkörpers 2 nahe dem äquatorialen Rand 12 ist als Verbindung zwischen den beiden Pfannenteilen 1 und 2 ein Kunststoffring 5 eingelegt, der, wie Fig. 3 zeigt, auf seinem Umfang einmal geschlitzt ist.

Die vergrösserte Darstellung der Fig. 2 lässt erkennen, dass der Ring 5 aussen eine ringförmige Nase 6 hat, die nach der Montage des Pfannenkörpers 2 in der Aussenschale 1 in eine Vertiefung 7 in der Wand des Innenhohlraumes der Aussenschale 1 einrastet und so eine feste, jedoch lösbare, Verbindung beider Pfannenteile 1 und 2 gewährleistet. Als Sicherung gegen eine Relativdrehung beider Teile 1 und 2 gegeneinander ist in der "Decke" des Hohlraumes der Aussenschale 1 eine Bohrung 8 vorgesehen, in die im Pass-Sitz ein Zapfen 9 an der Aussenseite des Pfannenkörpers 2 eingreift.

Zur Erleichterung der Montage des Pfannenkörpers 2 in der Aussenschale 1 sind zwei zylindrische Führungen 10 und 11 im Hohlraum der Aussenschale 1 bzw. auf der Aussenfläche des Pfannenkörpers 2 vorgesehen. Diese Führungen 10 und 11 sind dem Kunststoffring 5 polseitig vorgelagert und relativ zu ihm so angeordnet, dass sie beim Einschieben den Pfannenkörper 2 führen, ehe der Kunststoffring 5 mit dem unteren Rand 12 der Aussenschale, der für ein erleichtertes "Einfädeln" des Pfannenkörpers 2 eine Anschrägung 13 hat, in Kontakt tritt.

Weiterhin sind im äquatorialen Rand 12 der Aussenschale 1 Ausnehmungen 14, beispielsweise einander diametral gegenüberliegend vorgesehen, mit deren Hilfe ein nicht gezeigtes Instrument hinter die Nase 6 des Ringes 5 greifen kann, um - unter Zerstörung des Ringes 5 - den Pfannenkörper 2 aus der Aussenschale 1 zu lösen. Beim Wiedereinsetzen des Pfannenkörpers 2 wird dann ein neuer Ring 5 in die Nut 4 eingelegt, ehe der Pfannenkörper in die Aussenchale 1 eingeschoben wird.

**Patentansprüche**

1. Hüftgelenkspfanne mit einer Aussenschale (1) aus einem wenig verformbaren Werkstoff, in der ein ebenfalls wenig verformbarer Pfannenkörper (2) für die Aufnahme des Gelenkkopfes einer Femurkopfprothese dadurch lösbar verankert ist, dass am äusseren Umfang des Pfannenkörpers (2) in einer Nut (4) ein einfach geschlitzter Ring (5) eingelegt ist, der mit einer aus seinem Umfang vorspringenden ringförmigen Nase (6) in eine Ringnut (7) in der Innenwandung der Aussenschale (1) einschnappt, **dadurch gekennzeichnet,** dass der Ring aus Kunststoff ist und sich vollumfänglich auf den Grund der Nut (4) des Pfannenkörpers (2) abstützt, und dass Aussenschale (1) und Pfannenkörper (2) miteinander formschlüssig verbunden sind.

2. Hüftgelenkspfanne nach Anspruch 1, dadurch gekennzeichnet, dass Aussenschale (1) und Pfannenkörper (2) mit einer Verdrehsicherung (8, 9) versehen sind.

3. Hüftgelenkspfanne nach Anspruch 1, dadurch gekennzeichnet, dass Pfannenkörper (2) und Aussenschale (1) mit zylindrischen Führungen (10, 11) versehen sind, die dem Ring (5) polseitig vorgelagert sind.

**Claims**

1. An acetalbulum having an outer shell (1) made of a difficultly deformable substance, a cup (2) which is also made of a difficultly deformable substance being releasably secured in the acetabulum in order to receive the head of a femoral head prosthesis by a ring (5) formed with a single slit being introduced into a groove (4) in the outer periphery of the cup (2), the ring (5) snapping, by way of an annular nosing (6) which projects from its periphery, into an annular groove (7) in the outer shell inner wall, characterised in that the ring is made of plastics and is supported over its whole periphery on the base of the groove (4) in the cup (2), and the outer shell (1) and cup (2) are interconnected positively.

2. An acetabulum according to claim 1, characterised in that the outer shell (1) and cup (2) have means (8, 9) for preventing relative rotation between one another.

3. An acetabulum according to claim 1, characterised in that the cup (2) and outer shell (1) have cylindrical guides (10, 11) which are disposed nearer the pole than the ring (5) is.

**Revendications**

1. Cavité cotyloïde comportant une coque extérieure (1) réalisée dans un matériau peu déformable, dans laquelle est ancré de manière amovible un corps de cavité (2), peu déformable également, destiné au logement de la tête d'articulation d'une prothèse de tête de fémur, par le fait que sur le pourtour extérieur du corps de cavité (2), dans une rainure (4), est placé un anneau (5) simplement fendu qui, par un ergot (6) annulaire, dépassant de son pourtour, s'encliquette dans une rainure annulaire (7) de la paroi intérieure de la coque extérieure (1), caractérisée en ce que l'anneau est en matière synthétique et prend appui, sur tout son pourtour, sur le fond de la rainure (4) du corps de cavité (2), et en ce que la coque extérieure (1) et le corps de cavité (2) sont assemblés entre eux par concordance de forme.

2. Cavité cotyloïde selon la revendication 1, caractérisée en ce que la coque extérieure (1) et le corps de cavité (2) sont pourvus d'un blocage en rotation (8, 9).

3. Cavité cotyloïde selon la revendication 1, caractérisée en ce que le corps de cavité (2) et la coque extérieure (1) sont pourvus d'organes de guidage (10, 11) cylindriques qui sont placés devant l'anneau (5), sur le côté polaire.

## Fig. 1

## Fig. 2

## Fig. 3